Europäisches Patentamt

European Patent Office

Office européen des brevets

(19)

(11) Veröffentlichungsnummer : **0 054 724**
**B1**

(12) EUROPÄISCHE PATENTSCHRIFT

(45) Veröffentlichungstag der Patentschrift :
**15.05.85**

(21) Anmeldenummer : **81109349.1**

(22) Anmeldetag : **30.10.81**

(51) Int. Cl.⁴ : **C 11 D 1/28**, C 11 C 3/04,
C 07 C139/14, C 07 C143/90

(54) **Verfahren zur vereinfachten Gewinnung von hellfarbigen waschaktiven alpha-Sulfofettsäureestern.**

(30) Priorität : **19.12.80 DE 3047897**

(43) Veröffentlichungstag der Anmeldung :
**30.06.82 Patentblatt 82/26**

(45) Bekanntmachung des Hinweises auf die Patenterteilung : **15.05.85 Patentblatt 85/20**

(84) Benannte Vertragsstaaten :
**AT BE CH DE FR IT LI NL SE**

(56) Entgegenhaltungen :
DE-A- 1 443 995
DE-A- 1 468 032
DE-B- 1 234 709
DE-B- 1 246 717
DE-B- 1 248 645
US-A- 3 354 187

(73) Patentinhaber : **Henkel Kommanditgesellschaft auf Aktien
Postfach 1100 Henkelstrasse 67
D-4000 Düsseldorf-Holthausen (DE)**

(72) Erfinder : **Schmid, Karl, Dr.
Stifterstrasse 10
D-4020 Mettmann (DE)**
Erfinder : **Stein, Werner, Dr.
Am Ginsterweg 10
D-4000 Düsseldorf-Unterbach (DE)**
Erfinder : **Baumann, Horst, Dr.
Vogelwarte 17
D-5653 Leichlingen (DE)**

## Beschreibung

Die Erfindung geht von der allgemeinen Aufgabe aus, die bekannte Gewinnung waschaktiver α-Sulfofettsäureester aus Fetten und Ölen natürlichen Ursprungs, so zu vereinfachen und für die großtechnische Praxis wiederholbar auszugestalten, daß die Fettsäuren bzw. Fettsäuregemische pflanzlichen und/oder tierischen Ursprungs zur praktikablen Alternative bei der Herstellung moderner Waschmittelzusammensetzungen werden können. Auf dem Gebiet der Wasch- und Reinigungsmittel, insbesondere der Textil-Waschmittel, liegt bekanntlich bis heute das Schwergewicht bei Syntheseprodukten auf Erdöl-Basis, obwohl seit Jahrzehnten bekannt ist, daß hochwertige waschaktive Komponenten auch und gerade aus den Fettsäure-Triglyceriden natürlicher Ausgangsmaterialien gewonnen werden können.

So beschreibt die U.S.-A-2 195 187 α-Sulfofettsäuren und ihre Ester als waschaktive Substanzen. Sie werden durch Sulfonierung niederer Alkylester gesättigter höherer Fettsäuren mit Schwefeltrioxyd erhalten. Die als Ausgangsmaterial eingesetzten niederen Fettsäurealkylester werden durch Umesterung hydrierter Fette oder Öle mit einwertigen niederen Alkanolen, insbesondere Methanol, hergestellt.

Die Anmelderin hat sich in späteren Arbeiten intensiv mit dieser Klasse der waschaktiven α-sulfonierten Fettsäuren und entsprechenden Fettsäureestern sowie deren Salzen auseinandergesetzt. So beschreibt beispielsweise die DE-B-12 46 718 ein Verfahren zur Herstellung dieser Verbindungsklasse. Fettsäuren und Fettsäureester, die 6 bis 28 C-Atome im Fettsäurerest aufweisen und außer dem α-ständigen C-Atom des Fettsäurerestes keine weiteren sulfonierbaren bzw. sulfatierbaren Gruppen aufweisen und eine Jod-Zahl unterhalb 5 besitzen, werden mit einem Schwefeltrioxyd-Inertgas-Gemisch sulfoniert und das Umsetzungsprodukt neutralisiert. Ein mit alternativen Verfahrensmerkmalen, aber letztlich mit gleichen Mitteln arbeitendes Parallel-Verfahren zur Herstellung der gleichen Verbindungen ist in der DE-B-12 48 645 geschildert.

Eine der Hauptschwierigkeiten des hier betroffenen Gebiets liegt in der Farbinstabilität des Fettsäure enthaltenden Ausgangsmaterials im Sulfonierungsschritt. Es werden dunkel gefärbte, braunschwarze Rohprodukte erhalten, die zur Anwendung in Wasch- und Reinigungsmitteln zu hellfarbigen Produkten aufzuarbeiten sind. Die Farbe der Sulfonierungs-Rohprodukte ist zwar im bestimmten Ausmaß von den Arbeitsbedingungen abhängig, gleichwohl wird bis heute die technische Verwertung dieser an sich interessanten Rohstoff-Möglichkeit durch die folgende Gesetzmäßigkeit behindert: Je höher die Umsatzausbeute in der Sulfonierungsstufe (der Sulfonierungsgrad) getrieben wird, umso dunkler gefärbt ist das Reaktionsprodukt und umso größer werden die Schwierigkeiten, hellfarbige endprodukte zu gewinnen.

Die DE-C-12 62 265 schlägt zur Verringerung dieser Schwierigkeit und insbesondere zur verbesserten Steuerung der Sulfonierungsreaktion unter Vermeidung lokaler Überhitzungen, die zu unerwünschten Verfärbungen und Nebenreaktionen führen, vor, Gemische von 25-95 Gew.-% Alkylbenzolen und 5-75 Gew.-% gesättigter Fettsäureester zu sulfonieren. Hier sollen also beträchtliche Mengen an Alkylbenzolen als reaktiver Verdünner helfen, insbesondere die Farbschwierigkeiten zu bewältigen.

Die Bedeutung der Konstitution der zu sulfonierenden Fettsäuren bzw. Fettsäuregemische gilt in der Fachwelt als gesichertes Wissen. Es wird insbesondere gefordert, daß die in α-Position zu sulfonierenden Fettsäuren keine bzw. möglichst wenig Doppelbindungen sowie keine sonstigen reaktiven Gruppen — insbesondere Hydroxylgruppen — enthalten sollen. Durch Auswahl geeigneter Fette bzw. Öle wird dieses Problem auf die Beseitigung ungesättigter Bindungen im Fettsäuremolekül eingeschränkt. Diese Störstellen werden durch möglichst weitgehende Hydrierung des Ausgangsmaterials vor der Sulfonierung beseitigt. Die Literatur des Standes der Technik, fordert Jod-Zahlen unterhalb von 5, vorzugsweise unterhalb von 2. In den praktischen Beispielen wird mit weit niedrigeren Jod-Zahlen, z. B. solchen im Bereich von 0,1 bis 0,3 gearbeitet.

Auch die Abtrennung störender Begleitstoffe von den zu sulfonierenden Fettsäuren bzw. Fettsäureestern wird zur Verringerung des Verfärbungsproblems gefordert. Bei Fettsäuren und Fettsäureestern als Ausgangsmaterial wird empfohlen, von Destillaten auszugehen — siehe beispielsweise DE-B-12 48 645, Spalte 3, Abs. 2. Ist eine Destillation nicht möglich, beispielsweise bei Triglyceriden, werden andere Reinigungsschritte zur Entfernung von beispielsweise Eiweiß- und Schleimstoffen empfohlen.

Stets ist jedoch als abschließender Verfahrensschritt eine Bleiche der Rohsulfonsäure-Derivate nötig. Der Stand der Technik hat hier insbesondere zwei Verfahrenstypen entwickelt: Die saure Bleiche mit Wasserstoffperoxid — siehe hierzu DE-C-11 79 931 — oder eine Kombinationsbleiche, bei der sich an eine zunächst saure Wasserstoffperoxidbleiche die Neutralisierung des sulfonierten und teilgebleichten Gutes anschließt, woraufhin erneut mit Wasserstoffperoxid oder besser mit Hypochlorit eine abschließende Bleichstufe erfolgt — siehe hierzu DE-B-12 34 709.

Besondere Schwierigkeiten zum Problem der Verfärbung treten auf, wenn die Sulfonierung auf Umsatzausbeuten über 90 % oder gar auf Sulfonierungsgrade über 95 % getrieben werden soll. Die Lehre der DE-A-14 43 995 beschäftigt sich ausführlich mit den hier entstehenden Problemen. Das Schwefeltrioxyd hat nach den Angaben dieser Druckschrift auf gesättigte, von alkoholischen Hydroxylgruppen freie Fettsäureester eine stark zersetzende Wirkung, die bei der Herstellung hochsulfonierter Produkte mit einem Sulfonierungsgrad von wenigstens 90 %, vorzugsweise wenigstens 94 % und insbesondere wenigstens 96 %, unvermeidbar zu tiefdunkel verfärbten Sulfonierungs-Rohprodukten führt.

Die Erhöhung des Ausmasses der Sulfonierung in solche Bereiche ist aber nicht nur aus wirtschaftlichen Gründen interessant, weiterführende Gesetzmäßigkeiten fordern derart hohe Sulfonierungsgrade. So gilt : Erstersulfonate mit einem entsprechend niedrigen Sulfonierungsgrad führen zu Schwierigkeiten bei der üblichen Herstellung von Waschmittelkompositionen durch Zerstäuben. Beim Verarbeiten solcher Estersulfonate treten erfahrungsgemäß hohe Pluming-Werte auf. Weiterhin steht der Sulfonierungsgrad von Estersulfonaten in direktem Zusammenhang mit einem sich bei dieser Reaktion bildenden unerwünschten Nebenprodukt, der α-Sulfofettsäure. Diese nach der Neutralisation als Di-Natrium-Salz vorliegende Verbindung ist nur schlecht wasserlöslich und daher als Waschrohstoff untauglich. Eine Erhöhung des Sulfonierungsgrades von 90 % auf 96 % in den Estersulfonaten bewirkt eine Abnahme dieses unerwünschten Nebenproduktes von beispielsweise 25 % auf 16 %.

Die Lehre der DE-A-14 43 995, die sich mit der zuletzt besprochenen Problematik beschäftigt, schlägt zur Einschränkung der Verfärbung neben der Einhaltung bestimmter Temperaturen in der Sulfonierungsreaktion vor, in das Sulfonierungsprodukt so viel Wasser einzubringen, daß sich aus dem vorhandenen überschüssigen Schwefeltrioxyd und dem Wasser eine Schwefelsäure bildet, deren Konzentration bei Beginn der nachfolgenden Bleiche im Bereich von 100-20 Gew.-% $H_2SO_4$ liegt. Für das großtechnische Verfahren werden hierdurch jedoch neue Schwierigkeiten erzeugt, die eine beträchtliche Gefahrenquelle darstellen können. Die Viskosität des Sulfonierungsrohproduktes wird im starksauren Bereich schon durch geringste Wassermengen schwerwiegend beeinflußt. Schon der Zusatz von 2 % Wasserstoffperoxid in Form einer 35 %igen Lösung — und den damit zwangsläufig eingeführten Wassermengen — zum rohen Sulfonierungsprodukt mit der Kettenlänge C-16/C-18 führt zu einem steilen Viskositätsanstieg. Hierdurch entsteht im kontinuierlichen technischen Verfahren die Gefahr der Verstopfung von Rohrleitungen. Besonders kritisch ist dieser Viskositätsanstieg schon im Bereich des Zusatzes von 1,8 bis 2,5 Gew.-% Wasserstoffperoxid, berechnet auf die Rohsulfonsäure.

Aus dieser zu verfahrenstechnischen Schwierigkeiten führenden Besonderheit des rohen Sulfonierungsproduktes leitet sich darüberhinaus ab, daß die Bleiche mit Wasserstoffperoxid im sauren Bereich wenigstens bei Fettsäure-Alkylestern mit den Kettenlängen C-16/C-18, wie sie beispielsweise aus Talg und Palmöl gewonnen werden können, mit beträchtlichen Schwierigkeiten verbunden ist. Im einzelnen gilt : Werden beispielsweise Fettsäuremethylester, gewonnen durch Um- oder Veresterung, bei Temperaturen von 70-130 °C während ca. 10-90 Minuten sulfoniert, so fallen bei den angestrebten Sulfonierungsgraden von über 95 % tiefschwarze Reaktionsprodukte an. Zur Verwendung als Waschrohstoff bedarf es der Bleichung. Die Bleiche im alkalischen Medium mit Wasserstoffperoxid oder Natriumhypochlorit führt nicht zum ausreichenden Ergebnis. Selbst Hypochloritmengen von 3 Gew.-% — berechnet auf waschaktive Substanz — führen nur zu Klett-Farbzahlen im Bereich von 140. Höhere NaOCl-Mengen sind jedoch nicht mehr anwendbar, da das bei dieser Bleichreaktion entstehende Natriumchlorid zur unzulässigen Verdickung der neutralisierten Estersulfonatpaste führt. Es ist auch nicht möglich, Klett-Farbzahlen im gereinigten Produkt in Kauf zu nehmen, die über 50 bis 60 liegen. Unvollkommen gebleichte Estersulfonate bergen die Gefahr des Nachdunkelns in sich. Farbbeständigkeit ist jedoch für die Zerstäubungstrocknung beim Einsatz dieses Tensids in Waschmitteln unabdingbar.

Als bisher bestes Bleichverfahren für Rohestersulfonate ist die Verwendung von Wasserstoffperoxid im starksauren Bereich (pH = 0) beschrieben. Der Bleicheffekt ist hier besonders stark ausgeprägt. Es werden jedoch die zuvor geschilderten Gefahren des plötzlichen Viskositätsanstieges ausgelöst. Bei hoch-sulfonierten Estersulfonaten reichen selbst 2 Gew.-% Wasserstoffperoxid nicht aus, die Klett-Farbzahl von 50 einzustellen. Nach Neutralisation der Rohsulfonsäure muß daher mit Natriumhypochlorit nachgebleicht werden. Eine Verringerung der Bleichmittelmenge und gleichzeitige Erhöhung der Bleichzeit bei der sauren Bleiche mit Wasserstoffperoxid führt zu ungünstigeren Farben. Die Verwendung von Wasserstoffperoxid in größeren Mengen erfordert zusätzlich, daß in der neutralisierten Estersulfonatpaste erst nach Zerfall des bei der Bleichreaktion nicht abreagierten Wasserstoffperoxids eine Bleichung mit beispielsweise 0,5-1 Gew.-% Natriumhypochlorit vorgenommen werden kann, was ca. 24 Stunden in Anspruch nimmt. Bei noch vorhandenem $H_2O_2$ zeigt NaOCl eine stark verminderte Bleichwirkung.

Im Zusammenhang damit besteht eine Reihe weiterer Schwierigkeiten : durch den hohen Viskositätsanstieg bei Zusatz von beispielsweise 2 Gew.-% Wasserstoffperoxid zur Rohsulfonsäure ist es nicht möglich, bei der Neutralisation höhere Pastenkonzentrationen als 28 Gew.-% Waschaktivsubstanz herzustellen. Bei dieser Bleichmethode treten zudem Probleme mit der Schaumbildung auf, die technisch nur schwer beherrschbar sind, insbesondere führt der in die Rohsulfonsäure eingerührte Schaum zu weiterem Viskositätsanstieg.

Die vielgestaltigen und in unterschiedlichen Stufen des Gesamtverfahrens auftretenden Schwierigkeiten führen nach bisherigem Wissen zum aufgezwungenen Kompromiß zwischen Sulfonierung und Bleichung. Die in der Praxis optimal einstellbaren Sulfonierungsgrade liegen bei ca. 90 %.

Die Erfindung geht im engeren Sinn von der Aufgabe aus, Arbeitsbedingungen für den hier betroffenen Verfahrenstyp zu finden, mit denen sich ein ingesamt verbessertes Ergebnis erzielen läßt. Die Erfindung will insbesondere ermöglichen, Sulfonierungsgrade deutlich über 90 %, insbesondere über 95 %, einzustellen, gleichzeitig jedoch in einem technologisch sicheren Verfahren hellfarbig gebleichte Endprodukte mit Klett-Farbzahlen unter insbesondere 50 zu erhalten. Das Verfahren soll dabei generell für Fettsäuren des gesamten in der Natur vorliegenden C-Zahlbereichs gültig sein und soll insbesondere auch die sichere Aufarbeitung von C-16/C-18-Fraktionen ermöglichen.

Gegenstand der Erfindung ist dementsprechend ein Verfahren zur vereinfachten Gewinnung hellfarbiger waschaktiver Ester von Sulfofettsäuren bzw. ihrer Salze aus Fetten und Ölen pflanzlichen und/oder tierischen Ursprungs durch Umesterung bzw. Verseifung und Veresterung der gewünschtenfalls hydrierend gehärteten Fette bzw. Öle mit einwertigen $C_1$- bis $C_8$-Alkoholen, Abtrennung der gebildeten Fettsäureesterfraktion, soweit erforderlich deren Hydrierung, nachfolgende Sulfonierung sowie Bleichen des rohen Sulfonierungsproduktes, wobei dieses Verfahren dadurch gekennzeichnet ist, daß man in die Sulfonierungsstufe eine Fettsäureesterfraktion einbringt, aus der die begleitenden Fettsäureglyzeride bis auf einen Restgehalt von höchstens 1 Gew.-% — bezogen auf das zu sulfonierende Material — entfernt worden sind.

Die Lehre der Erfindung beruht auf der überraschenden Feststellung, daß es eines ganz bestimmten Reinigungsschrittes an dem in die Sulfonierungsstufe einzubringenden Fettsäureester bedarf, um die exzessive Verfärbung bei hohen Sulfonierungsgraden mit anschließenden Schwierigkeiten in der Bleiche zu vermeiden.

Der Lehre der Erfindung liegt die folgende Feststellung zugrunde : Werden die natürlichen Triglyceride pflanzlichen und/oder trierischen Ursprungs im konventionellen großtechnischen Verfahren der Umesterung mit monofunktionellen Alkoholen oder der Fettspaltung und anschliessenden Veresterung mit diesen Alkoholen unterworfen, so wird trotz destillativer Aufarbeitung als Hauptfraktion ein Fettsäurealkylester gewonnen, der noch einen geringen Gehalt an begleitenden Glyzeriden aufweist. « Begleitende glyzeride » sind im Sinne der erfindungsgemäßen Definition Mono-, Di- und/oder Triglyceride derjenigen Fettsäuren bzw. Fettsäuregemische, die als Hauptprodukt in den Monoalkylester umgewandelt worden sind, in einer Restmenge aber auch noch in Form solcher Glyzeride als begleitende Verunreinigung in dem zu sulfonierenden Material vorliegen. Der Gehalt an diesen begleitenden Glyzeriden liegt üblicherweise in der Größenordnung von 2 bis maximal 5 Gew.-%, im allgemeinen macht er etwa 2 bis 3 Gew.-% der Fettsäure esterfraktion aus. Es sind verschiedene Wege vorstellbar, wie trotz Destillation der Hauptfraktion ein solcher Glyzeridgehalt in das Produkt übergeführt wird, eine Klärung dieser Frage ist für die Erfindung jedoch nicht wesentlich.

Entscheidend ist vielmehr, daß erfindungsgemäß erkannt wurde, daß dieser geringe Gehalt an begleitenden Glyzeriden eine offenbar überproportionale Rolle bei der Ausbildung der unerwünschten starken Verfärbung während der Sulfonierungsreaktion spielt. So zeigt sich überraschenderweise, daß die teilweise, vorzugsweise überwiegende Entfernung dieser begleitenden Glyzeride zu einer solchen Verringerung der verfärbten Nebenprodukte in der Sulfonierungsreaktion führt, daß jetzt die technische Lösung der geschilderten Aufgabenstellung der Erfindung gelingt.

Der maximale Restgehalt der begleitenden Glyzeride in der in die Sulfonierungsstufe einzusetzenden Fettsäureesterfraktion liegt bei 1 Gew.-%, bezogen auf diese Fettsäureesterfraktion. Es kann bevorzugt sein, mit Restgehalten der begleitenden Glyzeride von höchstens 0,5 Gew.-% oder vorzugsweise sogar von höchstens 0,3 Gew.-% zu arbeiten. Es können auch noch niedrigere Restgehalte der begleitenden Glyzeride eingestellt werden, beispielsweise solche von etwa 0,2 Gew.-% oder gar 0,1 Gew.-%. Bevorzugt werden die begleitenden Glyzeride vollständig beseitigt. Je nach den im folgenden noch geschilderten Parametern des erfindungsgemäßen Verfahrens und insbesondere der Beschaffenheit des jeweils als erstes Ausgangsmaterial eingesetzten pflanzlichen und/oder tierischen Naturstoffes ist die Lösung der eingangs geschilderten Problematik durch Bestimmung und Einstellung des maximal zulässigen Restgehalts der begleitenden Glyzeride möglich.

Die Verringerung und Kontrolle des zulässigen Restgehaltes der begleitenden Glyzeride ist insbesondere auf destillativem Wege möglich. Wird die Steuerung dieses Gehalts störender Glyzeride auf destillativem Wege durchgeführt, so gilt das Folgende : Während im üblichen technologischen Verfahren die destillative Auftrennung des Reaktionsproduktes aus der Fett- bzw. Öl-Umesterung oder -Spaltung und anschließender Veresterung mit monofunktionellen Alkoholen zu der geschilderten Beimengung der begleitenden Glyzeride führt, lassen sich durch eine erneute Destillation die Glyzeride aus dieser Fraktion abtrennen. Dabei kann eine für die Zwecke der Erfindung befriedigende Abtrennung schon durch eine einfache Über-Kopf-Destillation erfolgen. Arbeitet man beispielsweise mit einer entsprechenden Fettsäuremethylesterfraktion, so kann die erfindungsgemäß gewünschte Reinigung durch die Über-Kopf-Destillation bei einer Sumpftemperatur bis zu etwa 280 °C bei 1 mbar erfolgen. Gewünschtenfalls kann eine stärkere Trennwirkung durch Einschalten einer Fraktionierzone erzielt werden. Daß in dieser zweiten Destillationsstufe die wenigstens weitgehende Abtrennung der begleitenden Glyzeride gelingt, während in der vorgängigen ersten Destillationsstufe die geschilderte Beimengung der begleitenden Glyzeride das übliche Ergebnis ist, liegt möglicherweise daran, daß in der ersten Destillationsstufe stets freies Glyzerin aus der Fett- bzw. Öl-Spaltung oder Umesterung vorliegt, da das Glyzerin bei der üblichen Wasserwäsche nur unvollständig aus dem Produkt der Umesterung ausgewaschen wird.

Das aus der anschließenden Sulfonierung gewonnene α-Sulfofettsäureester-Rohprodukt wird zum gewünschten hellfarbigen Endprodukt gebleicht. Bevorzugt erfolgt die Bleiche mit Wasserstoff-Peroxid bzw. $H_2O_2$ liefernden Verbindungen und/oder Hypochlorit. Im erfindungsgemäßen Verfahren ist es möglich, die Bleichung mit nur einem der genannten Bleichmittel durchzuführen oder aber eine Kombinationsbehandlung einzusetzen. Im einzelnen gilt :

1. Bleichmethode — Alkalisches Bleichverfahren mit Natriumhypochlorit

Die Rohsulfonsäure des Estersulfonats wird, wie üblich, neutralisiert, siehe hierzu die eingangs geschilderten Druckschriften der Anmelderin aus dem Stand der Technik. Da Estersulfonat-Pasten bei einem pH-Wert kleiner 6 sehr viskos werden, bei einem pH-Wert größer 10 die Gefahr der Esterspaltung besteht, wird die Neutralisation zweckmäßig durch gleichzeitiges kontinuierliches Zusammenführen von Rohsulfonsäure und Natronlauge im pH-Bereich von 6,5-10 durchgeführt. Für die Bleichung mit Natriumhypochlorit bestehen zwei Möglichkeiten : a) Man benutzt die Alkalität der Hypochloritlösung bereits bei der Neutralisation. Hier wird also die Neutralisation durch kontinuierliches gleichzeitiges Zusammenführen von Natronlauge, Rohsulfonsäure und Natriumhypochloritlösung zweckmäßig im pH-Bereich von 7 bis 9 vorgenommen. b) Die Rohsulfonsäure wird zunächst neutralisiert und erst nachträglich mit Natriumhypochlorit gebleicht.

Die für eine Klett-Farbzahl von 50 oder weniger notwendige Bleichmittel-Menge an Natriumhypochlorit beträgt — je nach dem Ausmaß der erfindungsgemäßen Vorreinigung — 0,1 bis 3 Gew.-% Natriumhypochlorit — bezogen auf zu bleichendes Material. Üblicherweise liegt die Natriumhypochlorit-Menge hier zwischen 1 und 3 Gew.-%, wobei Mengen von 2 bis 3 Gew.-% besonders zweckmäßig sein können. Die Verwendung größerer Mengen an Hypochlorit ist nicht ausgeschlossen, kann allerdings technische Schwierigkeiten bringen, da die dabei entstehende Kochsalzmenge zur starken Verdickung der Estersulfonat-Paste führen kann.

Die Temperatur der Hypochlorit-Bleiche liegt bevorzugt im Temperaturbereich von etwa 50-70 °C, insbesondere im Bereich von 55-65 °C. Die Dauer der Bleichbehandlung liegt im allgemeinen im Bereich von 1 bis 10 Stunden, vorzugsweise im Bereich von 2 bis 4 Stunden. Als optimal hat sich zur Erreichung einer Klett-Farbzahl von 50 und darunter eine Bleichbehandlung für ca. 3 Stunden bei 60 °C ± 5 °C erwiesen.

Höhere Temperaturen führen in Richtung auf Farbverschlechterungen, niedrigere Temperaturen fordern zu lange Bleichzeiten. Ein großer Vorteil dieser Bleiche besteht darin, daß sich die Produkte mit Klett-Farbzahl von 50 als ausreichend farbstabil in der weiteren Verarbeitung erweisen. So hat beispielsweise ein wie zuvor angegebenes Material eine Klett-Farbzahl von 40 (Bleichung der Estersulfonat-Paste bei 60 °C/3 Stunden), während nach 24-stündiger Lagerung bei 60 °C die Klett-Farbzahl nur auf 42 angestiegen ist. Unvollkommen gebleichte Estersulfonatpasten sind demgegenüber nicht farbstabil, wobei die Geschwindigkeit des Anstiegs der Klett-Farbzahl im direkten Zusammenhang zu dem bei der Bleichung erreichten Farbwert steht.

Ein weiterer Vorteil der hier geschilderten Bleichmethode besteht darin, daß damit erstmalig Estersulfonat-Pasten mit einem Gehalt an waschaktiver Substanz über 28 Gew.-% einstellbar sind. So ist es beispielsweise möglich, Gehalte an Waschaktivsubstanz im Bereich von 30-40 Gew.-% einzustellen.

2. Bleichmethode — Saures Bleichverfahren mit Wasserstoffperoxid bzw. kombiniertes Verfahren

Die erfindungsgemäß vorgesehene Abtrennung der begleitenden Fettsäureglyzeride aus der zu sulfonierenden Fettsäureester-Fraktion ermöglicht die Verwendung beschränkter Mengen an Wasserstoffperoxid, wobei sowohl die alleinige Verwendung von Wasserstoffperoxid als auch dessen Kombination mit einem anschliessenden zweiten Bleichschritt, insbesondere der Hypochlorit-Bleiche, möglich ist. Die Wasserstoffperoxid-Bleiche wird im sauren Bereich vorgenommen. Vorzugsweise wird das rohe Sulfonierungsprodukt unmittelbar mit Wasserstoffperoxid versetzt. In der bevorzugten Ausführungsform dieses Bleichverfahrens wird mit weniger als 2 Gew.-% $H_2O_2$, berechnet als 100 %iges $H_2O_2$ und bezogen auf das rohe Sulfonierungsprodukt, gearbeitet. Mengen bis zu 1,5 Gew.-% $H_2O_2$ und insbesondere solche im Bereich von 0,5 bis 1,5 Gew.-% $H_2O_2$ können bevorzugt sein.

Das erfindungsgemäße Verfahren ermöglicht es, Klett-Farbzahlen im erwünschten Bereich alleine mit einer Behandlung des rohen sulfonierungsproduktes mittels $H_2O_2$ einzustellen. Wird mit beispielsweise 1 Gew.-% $H_2O_2$ (als 100 %iges Material, berechnet und bezogen auf das rohe Sulfonierungsprodukt) gearbeitet, so kann in mehrfacher Weise von Vorteilen Gebrauch gemacht werden. Die Probleme der Schaumentwicklung und der $H_2O_2$-Restmengen nach erfolgter Bleichung sind deutlich entschärft. Die Herstellung weitgehend kochsalzfreier Estersulfonat-Pasten wird möglich. Das wirkt sich wiederum günstig auf die einstellbaren Feststoffgehalte in dem Fertigprodukt aus.

Besonders wirkungsvoll kann die kombinierte Anwendung von Wasserstoff-Peroxid und Hypochlorit sein. So kann in einer ersten Verfahrensstufe das rohe Sulfonierungsprodukt mit 0,5-1,5 Gew.-% $H_2O_2$, insbesondere 1 Gew.-% $H_2O_2$ (jeweils als 100 %iges Material berechnet) vorgebleicht werden, woraufhin sich eine Nachbleichung mit 0,5-1,5 Gew.-% NaOCl, insbesondere 1 Gew.-% NaOCl, anschließt. Zur Hypochlorit-Bleiche gelten hier die zuvor gemachten Angaben zu 1.

Einzelheiten zur Durchführung der sauren Bleiche oder der mehrstufigen Kombinationsbleiche finden sich beispielsweise in den genannten DE-C-11 79 931, 12 34 709 und der DE-A-14 43 995. So gilt beispielsweise, daß die $H_2O_2$-Bleiche bei Temperaturen nicht über 100 °C, insbesondere unterhalb 80 °C, durchgeführt wird. Die Dauer des Bleichens variiert mit dem Ausgangsmaterial, der Wasserstoffsuperoxidmenge und der Temperatur. Sie kann im Bereich von 15 Minuten bis zu einigen Stunden, beispielsweise 5 bis 10 Stunden, liegen.

Im einzelnen gelten zu den zahlreichen Stufen des erfindungsgemäß betroffenen Gesamtverfahrens die Angaben des zitierten Standes der Technik, die hier nur kurz wiederholt seien :

Die als Ausgangsmaterialien eingesetzten Öle und/oder Fette können Pflanzen, Land- oder Wassertieren entstammen. Ihre Fettsäurereste liegen überwiegend innerhalb des C-Zahlbereichs von 8 bis 18, wobei sowohl Fette mit bevorzugt 10 bis 14 Kohlenstoffatomen in den Fettsäureresten als solche mit bevorzugt 16 bis 18 Kohlenstoffatomen in den Fettsäureresten gleichermaßen geeignet sind. Sulfatierbare oder sulfonierbare Gruppen, die — abgesehen von α-ständigen Wasserstoffatom des Fettsäurerestes — in diesen Fettsäuren bzw. deren Estern nicht vorhanden sein sollen, sind beispielsweise Doppelbindungen oder alkoholische Hydroxylgruppen. Ursprünglich vorliegende Doppelbindungen können durch Hydrierung beseitigt werden. Es ist dabei möglich, diese Hydrierung am Fett- bzw. Öl-Ausgangsmaterial vorzunehmen. Bevorzugt wird jedoch der nach der Glyzeridspaltung erhaltene Fettsäurealkylester hydrierend gesättigt. Diese Hydrierung erfolgt in an sich bekannter Weise. Die Produkte der Hydrierung sollen Jodzahlen unter 2, vorzugsweise unter 1 und insbesondere im Bereich von 0,5 oder darunter, aufweisen. Bevorzugte Ausgangsmaterialien für die anschließende Sulfonierung sind Talgfettsäuremethylester und Palmölfettsäuremethylester.

Die erfindungsgemäße Abtrennung der begleitenden Fettsäureglyzeride aus dem Ester der Fettsäuren mit den monofunktionellen Alkoholen kann vor oder nach der Hydrierung dieser Fettsäureester erfolgen. Bevorzugt wird die Entfernung der begleitenden Fettsäureglyzeride nach der Hydrierung des Fettsäurealkylesters vorgenommen.

Das so vorbereitete Material wird dann in an sich bekannter Weise zum Beispiel bei Temperaturen von 70 bis 130 °C in einem Fallfilmreaktor mit einem Gemisch von gasförmigen Schwefeltrioxyd und Inertgas während eines Zeitraumes von 10 bis 90 Minuten sulfoniert. Der Sulfonierungsgrad beträgt vorzugsweise mehr als 90 %, insbesondere mehr als 92 %, und in der Regel mehr als 94 %. Sulfonierungsgrade von 95 % und darüber sind besonders bevorzugt.

Die anschließende Bleiche erfolgt in einer der zuvor angegebenen Formen. Klett-Farbzahlen unter 60 und insbesondere bis zu 50 werden hierbei erfindungsgemäß eingestellt. Das gebleichte Gut kann — soweit noch erforderlich — zur neutralen Paste des pH-Bereichs von 7-9, insbesondere 7,5-8,5, neutralisiert werden.

Die folgenden Beispiele erläutern die Erfindung. Damit im Zusammenhang wird versucht, aufklärung für den überraschenden Effekt zu finden, der der erfindungsgemäßen Lehre zugrunde liegt. Hier hat sich das Folgende gezeigt :

Die Gegenwart von Fettsäureglyzeriden in der Sulfonierungsstufe ist nicht grundsätzlich schädlich. Werden dem zu sulfonierenden Material Glyzeride von nicht reaktiven Fettsäuren, beispielsweise gesättigten Fettsäuren, zugesetzt, so findet keine zusätzliche Beeinträchtigung der Klett-Farbzahl statt. Stark färbend wirken jedoch zugesetzte Spuren des Glyzerids der Rizinolsäure oder auch der Hydroxystearinsäure. Eine mögliche Erklärung für die Wirkungen des erfindungsgemäßen Vorschlages könnte im Folgenden liegen : Bei der Verwendung natürlicher Fettsäurequellen, wie sie erfindungsgemäß vorgesehen ist, muß stets davon ausgegangen werden, daß durch mehr oder weniger starkes Ranzigwerden des Ausgangsmaterials in geringem Umfang Oxydationsvorgänge, insbesondere an ungesättigten Fettsäuremolekülen, stattgefunden haben. Der Anteil des ranzigen Materials ist prozentual gesehen außerordentlich gering, in der Sulfonierungsstufe macht er sich aber möglicherweise stark bemerkbar. Durch die Entfernung der mitgeschleppten Glyzerid-Fraktion gelingt erfindungsgemäß die überproportionale positive Beeinflussung der Verfärbung in der Sulfonierungsstufe. Es sind also nur diese aus dem ursprünglichen Ausgangsmaterial mitgeschleppten Anteile in Glyzeridbindung, die störend wirken. Der gezielte Zusatz oder die Gegenwart von farbstabilen Glyzeriden stört im Sinne des erfindungsgemäßen Verfahrens nicht.

In den folgenden Beispielen bedeuten Prozentangaben Gew.-%, soweit nicht anders angegeben ist.

Vergleichsversuch 1

Als Einsatzmaterial diente ein Talgfettsäuremethylester, gewonnen durch Umesterung von Talg mit Methanol mit anschließender Destillation und Härtung, mit folgenden Kennzahlen : JZ 0,3 ; OHZ = 1,1 ; SZ = 0,5 ; VZ = 195,8.

573 g ($\hat{=}$ 2 Mol) dieses Materials wurden in einem auf 80 °C erwärmten Standzylinder durch 65 minütiges Einblasen eines 5 Vol.-%igen

SO$_3$/Luft-Gemisches, das eine Gesamtmenge von 208 g ($\hat{=}$ 2,6 Mol) SO$_3$ enthielt, und einer anschließenden Nachreaktionszeit von 15 Minuten sulfoniert. Die Menge der so erhaltenen Rohsulfonsäure, die einen Sulfonierungsgrad von 98 % aufwies, wurde halbiert. Eine Hälfte davon wurde sofort durch gleichzeitiges Zusammenführen von Rohsulfonsäure mit Natronlauge im pH-Bereich von 6,5 bis 8 zu einer ca. 25 Gew.-% Sulfonierungsprodukt enthaltenden wäßrigen Paste neutralisiert, die anschließend mit 15,4 bzw. 23,1 Gew.-% einer 13 %igen wäßrigen NaOCl-Lösung — bezogen auf Sulfonierungsprodukt — bei 60 °C während einer Stunde gebleicht wurde. Eine in bezug auf Sulfonierungsprodukt 5 %ige wäßrige Lösung, eingestellt auf pH 7 ; zeigte im Klettphotometer (Modell 800-3 der Fa. Klett-Summerson) mit Blaufilter (420 nm) in einer Klett-Rundglas-Küvette eine Klettfarbzahl von 210 bzw. 180.

Mit der anderen Hälfte wurde wie folgt verfahren : Die Rohsulfonsäure wurde zunächst durch Zugabe von 2,86 Gew.-% einer 35 %igen wäßrigen H$_2$O$_2$-Lösung bei 55 °C während 15 Minuten gebleicht, anschließend wie beschrieben mit Natronlauge neutralisiert und mit 7,69 Gew.-% einer 13 %igen wäßrigen NaOCl-Lösung — bezogen auf Sulfonierungsprodukt — bei 60 °C während einer Stunde gebleicht. Die so erhaltene Paste hatte eine Klettzahl (5 % Sulfonierungsprodukt, Blaufilter, pH 7) von 130.

**Vergleichsversuch 2**

Ein in analoger Weise wie im Vergleichsversuch 1 hergestellter Talgfettsäuremethylester wurde zusätzlich nochmals gehärtet und hatte folgende Kennzahlen : JZ = 0,08 ; OHZ = 1,1 ; SZ = 0,4 ; VZ = 195,8.

Dieses Material wurde, wie in Vergleichsversuch 1 beschrieben sulfoniert (Sulfonierungsgrad 98 %), neutralisiert und gebleicht, wobei als Bleichmittel für die eine Hälfte nur NaOCl, für die andere eine Kombination von Wasserstoffperoxid und NaOCl verwendet wurde. Die so erhaltenen Klettfarbzahlen betrugen 220 (2 Gew.-% NaOCl) bzw. 160 (3 Gew.-% NaOCl) und 160 (1 Gew.-% H$_2$O$_2$ und 1 Gew.-% NaOCl). Die Gew.-% Bleichmittel sind jeweils auf Sulfonierungsprodukt berechnet, wobei das Bleichmittel als 100 %ig betrachtet wird.

**Beispiel 1**

320 kg eines wie in Vergl. Vers. 1 hergestellten Talgfettsäuremethylesters wurden zusätzlich destilliert, wobei bis zu einer Sumpftemperatur von 246 °C/1 mbar 309 kg als Destillat übergingen. Der Rückstand von 3,4 Gew.-% des Einsatzmaterials bestand überwiegend aus Mono-, Di- und Triglyceriden der im Talg vertretenen Fettsäuren. 3 Gew.-% dieses Rückstandes wurden als Hydroxystearin- und Hydroxyölsäureglycerid identifiziert.

Das erhaltene Talgfettsäuremethylester-Destillat mit den Kennzahlen : JZ = 0,3 ; OHZ = 0,7 ; SZ = 0,7 ; VZ = 196 wurde, wie in Vergl. Vers. 1 beschrieben, sulfoniert (Sulfonierungsgrad 98 %) und gebleicht. Die erhaltenen Klettzahlen betrugen 96 (2 Gew.-% NaOCl) und 55 (3 Gew.-% NaOCl) bzw. 35 (1 Gew.-% H$_2$O$_2$ und 1 Gew.-% NaOCl).

**Vergleichsversuch 3**

Als Einsatzmaterial diente ein Talgfettsäuremethylester, gewonnen durch Spaltung des Talgs, Auswaschen des Glyzerins mit nachfolgender Destillation und Veresterung der Talgfettsäure mit anschließender Härtung.

Dieser Talgfettsäuremethylester mit den Kennzahlen : JZ = 0,4 ; OHZ = 1,1 ; SZ = 0,4 ; VZ = 195 wurde wie in Vergleichsversuch 1 beschrieben sulfoniert (Sulfonierungsgrad 96 %) und gebleicht. Die erhaltenen Klettzahlen betrugen bei einer Bleichzeit von 1 Stunde bei 60 °C 125 (2 Gew.-% NaOCl) und 90 (3 Gew.-% NaOCl) bzw. 68 (1 Gew.-% H$_2$O$_2$ und 1 Gew.-% NaOCl), wobei das Bleichmittel als 100 %ig gerechnet wird und die Gew.-% sich auf den Gehalt an sulfoniertem Produkt beziehen.

**Beispiel 2**

Das in Vergleichsversuch 3 beschriebene Einsatzmaterial wurde, wie in Beispiel 1 beschrieben, zusätzlich durch Destillation gereinigt, wobei ein Destillationsrückstand von 2,3 Gew.-% anfiel. 1,8 Gew.-% dieses Rückstandes wurden als Hydroxystearin- und Hydroxyölsäureglycerid identifiziert.

Das Destillat mit den Kennzahlen : JZ = 0,3 ; OHZ = 1,1 ; SZ = 0,4 ; VZ = 195 wurde in gleicher Weise wie in Vergleichsversuch 1 sulfoniert (Sulfonierungsgrad 96 %) und gebleicht. Die erhaltenen Klettzahlen betrugen bei einer Bleichzeit von 1 Stunde bei 60 °C 52 (2 Gew.-% NaOCl) und 40 (3 Gew.-% NaOCl) bzw. 18 (1 Gew.-% H$_2$O$_2$ und 1 Gew.-% NaOCl), wobei das Bleichmittel als 100 %ig gerechnet wird und die Gew.-% sich auf den Gehalt an sulfoniertem Produkt beziehen.

**Beispiel 3**

Zu dem für das Beispiel 1 beschriebenen Einsatzmaterial, einem destillierten Talgfettsäuremethylester, wurden je 1 Gew.-% Behensäuremethylester, Glyzerinmonostearat und Glyzerintristearat zugesetzt. Dieses Gemisch wurde wieder, wie in Beispiel 1 beschrieben, sulfoniert (Sulfonierungsgrad 96 %) und gebleicht. Nach einer Bleichzeit von 1 Stunde mit Natriumhypochlorit erhielt man Pasten mit einem Gehalt an sulfoniertem Produkt von 26 Gew.-%, die Klettzahlen von 43 (2 Gew.-% NaOCl) bzw. 30 (3 Gew.-% NaOCl) aufwiesen.

**Beispiel 4**

Zu dem für das Beispiel 1 verwendeten destillierten Talgfettsäuremethylester wurden 2 Gew.-% Glyzerintristearat zugesetzt und dieses Gemisch nach Beispiel 1 sulfoniert (Sulfonierungsgrad 96 %) und gebleicht. Die nach einer Stunde Bleichzeit bei 60 °C mit Natriumhypochlorit als Bleichmittel erhaltenen Klettzahlen waren 65 (2 Gew.-% NaOCl) bzw. 47 (3 Gew.-% NaOCl).

Beispiel 5

Zu dem für das Beispiel 1 verwendeten destillierten Talgfettsäuremethylester wurden 2 Gew.-% Palmitinsäuremonoglycerid zugemischt und diese Mischung nach Vergleichsversuch 1 sulfoniert (Sulfonierungsgrad 96 %) und gebleicht. Die nach einer Stunde Bleichzeit bei 60 °C mit Natriumhypochlorit als Bleichmittel erhaltenen Klettzahlen waren 42 (2 Gew.-% NaOCl) bzw. 30 (3 Gew.-% NaOCl).

Beispiel 6

Wie in Beispiel 5 wurden zu dem für das Beispiel 1 verwendeten destillierten Talgfettsäuremethylester 3 Gew.-% eines Gemisches aus Ölsäuremono-, -di- und -triglycerid, das zu etwa 10 % noch die entsprechenden Glyzeride der Linol- und zu 1 % die der Linolensäure enthielt, zugesetzt und dieses Produktgemisch auf eine JZ von kleiner 0,1 gehärtet. Nach der in Beispiel 1 beschriebenen Sulfonierung (Sulfonierungsgrad 96 %) und Bleichung mit Natriumhypochlorit erhielt man nach einer Bleichzeit von 1 Stunde Klettzahlen von 36 (2 Gew.-% NaOCl) bzw. 23 (3 Gew.-% NaOCl).

Vergleichsversuch 4

Wie in Beispiel 6 wurden zu dem für das Beispiel 1 verwendeten destillierten Talgfettsäuremethylester 3 Gew.-% eines Gemisches aus Ricinolsäuremono-, -di- und -triglycerid zugesetzt und dieses Produktgemisch auf eine JZ von kleiner 0,1 gehärtet. Nach der in Beispiel 1 beschriebenen Sulfonierung (Sulfonierungsgrad 93 %) und Bleichung mit Natriumhypochlorit erhielt man nach einer Bleichzeit von 1 Stunde Klettzahlen von 425 (2 Gew.-% NaOCl) bzw. 270 (3 Gew.-% NaOCl).

Vergleichsversuch 5

Durch das im Beispiel 6 verwendete Gemisch von Ölsäuremono-, -di- und -triglycerid, das zu 10 Gew.-% die entsprechenden Glyzeride der Linolsäure und zu 1 Gew.-% die der Linolensäure enthielt, wurde während 6 Stunden bei 100 °C Luft durchgeleitet. Dadurch erhöhte sich die OHZ des Gemisches von 220 auf 240.

Von diesem Gemisch wurden 3 Gew.-%, wie in Beispiel 6, zu dem für das Beispiel 1 verwendeten destillierten Talgfettsäuremethylester zugesetzt und dieses so erhaltene Produktgemisch gehärtet (Kennzahlen : JZ 0,05 ; SZ = 1,4 ; OHZ = 7,0 ; VZ = 196). Nach der in Vergleichsversuch 1 beschriebenen Sulfonierung (Sulfonierungsgrad 96 %) und Bleichung mit Natriumhypochlorit erhielt man nach einer Bleichzeit von 1 Stunde Klettzahlen von 180 (2 Gew.-% NaOCl) und 130 (3 Gew.-% NaOCl).

Vergleichsversuch 6

Als Einsatzmaterial diente ein Talgfettsäuremethylester, gewonnen durch Umesterung von Talg mit Methanol mit anschließender Destillation und Härtung, mit folgenden Kennzahlen : JZ 0,3 ; OHZ = 1,1 ; SZ = 0,5 ; VZ = 195,8.

42,9 kg pro Stunde dieses Materials wurden kontinuierlich in einem Fallfilmreaktor mit 5 Vol.-% $SO_3$/Luft und einem $SO_3$/Ester-Verhältnis von 1,2 : 1 bei 90 °C während ca. 25 Minuten sulfoniert (Sulfonierungsgrad : 90,4 %), im pH-Bereich 6 bis 9 mit Natronlauge neutralisiert und mit 3 Gew.-% NaOCl (berechnet als 100 %ig und bezogen auf das sulfonierte Produkt) während 4 Stunden bei 60 °C gebleicht. Die Klettzahl der neutralen Estersulfonatpaste betrug 95 (5 %ige Estersulfonatlösung, Blaufilter, pH 7).

Beispiel 7

4 Tonnen des für Vergleichsversuch 6 verwendeten Einsatzmaterials wurden zusätzlich bis zu einer Sumpftemperatur von 250 °C bei 1 mbar destilliert. Der Destillationsrückstand betrug ca. 3 Gew.-%.

Das destillierte Material wurde gemäß Vergleichsversuch 6 kontinuierlich sulfoniert (Sulfonierungsgrad 96 %) und mit 2,7 Gew.-% Natriumhypochlorit gebleicht. Nach einer Bleichzeit von 4 Stunden betrug die Klettzahl der neutralen Estersulfonatpaste (30 %ig) 40 (5 %ige Estersulfonatlösung, Blaufilter, pH 7).

Beispiel 8

42,9 kg pro Stunde des in Beispiel 7 für die Sulfonierung verwendeten Einsatzmaterials wurden wie dort beschrieben sulfoniert, die Rohsulfonsäure mit 2,86 Gew.-% 35 %iger Wasserstoffperoxidlösung (1 Gew.-%, gerechnet als 100 %ig) vorgebleicht, dann neutralisiert und mit 1 Gew.-% NaOCl (gerechnet als 100 %ig, bezogen auf das zu sulfonierende Material) nachgebleicht. Die Klettzahl der neutralen Estersulfonatpaste (29 %ig) betrug 50 (5 %ige Estersulfonatlösung, Blaufilter, pH 7).

**Patentansprüche**

1. Verfahren zur vereinfachten Gewinnung hellfarbiger waschaktiver Ester von α-Sulfofettsäuren bzw. ihrer Salze aus Fetten und Ölen pflanzlichen und/oder tierischen Ursprungs durch Umesterung bzw. Verseifung und Veresterung der gewünschtenfalls hydrierend gehärteten Fette bzw. Öle mit einwertigen $C_1$- bis $C_8$-Alkoholen, Abtrennung der gebildeten Fettsäureester- fraktion, soweit erforderlich deren Hydrierung, nachfolgende Sulfonierung sowie Bleichen des rohen Sulfonierungsproduktes, dadurch gekennzeichnet, daß man in die Sulfonierungsstufe eine Fettsäureesterfraktion einbringt, aus der die begleitenden Fettsäureglyzeride bis auf einen Restgehalt von höchstens 1 Gew.-% — bezogen auf das zu sulfonierende Material — entfernt worden sind.

2. Verfahren nach Anspruch 1, dadurch gekennzeichnet, daß der Restgehalt der begleitenden Glyzeride in der Sulfonierungsstufe nicht mehr als etwa 0,5 Gew.-%, vorzugsweise nicht mehr als etwa 0,3 Gew.-%, beträgt.

3. Verfahren nach Ansprüchen 1 und 2, dadurch gekennzeichnet, daß man bei der Trennung des Reaktionsgemisches aus der Umesterung bzw. Verseifung und Veresterung der Fette und Öle destillativ eine Fettsäureesterfraktion mit einem Gehalt begleitender Glyzeride von etwa 2 bis 5 Gew.-%, insbesondere etwa 2 bis 3 Gew.-%, gewinnt, diese Fraktion durch erneute Destillation vom größeren Anteil ihrer Glyzeride befreit und sulfoniert.

4. Verfahren nach Ansprüchen 1 bis 3, dadurch gekennzeichnet, daß die zweite Destillation der Fettsäureesterfraktion durch Über-Kopf-Destillation bis zu einer Sumpftemperatur von etwa 280 °C bei 1 mbar erfolgt.

5. Verfahren nach Ansprüchen 1 bis 4, dadurch gekennzeichnet, daß bis zu einem Sulfonierungsgrad von wenigstens 92 %, vorzugsweise von wenigstens 95 %, sulfoniert wird.

6. Verfahren nach Ansprüchen 1 bis 5, dadurch gekennzeichnet, daß das α-Sulfofettsäureester-Rohprodukt einer Bleiche mit Wasserstoff-Peroxid bzw. $H_2O_2$ liefernden Verbindungen und/oder Hypochlorit zu Produkten mit einer Klett-Farbzahl von höchstens 60, vorzugsweise von 50 oder weniger, unterworfen wird, wobei zweckmäßigerweise (a) entweder eine Kombinationsbleiche mit $H_2O_2$ im sauren Bereich und nachfolgender Hypochlorit-Behandlung im pH-Bereich von 6,5 bis 10 oder (b) ausschließlich eine Hypochlorit-Behandlung im ph-Bereich von 6,5-10 erfolgt.

7. Verfahren nach Anspruch 6, dadurch gekennzeichnet, daß bei der Kombinationsbleiche gemäß (a) 0,5 bis 1,5 Gew.-% $H_2O_2$ — berechnet als 100 %iges $H_2O_2$ und bezogen auf das sulfonierte Material —, vorzugsweise 1 Gew.-% $H_2O_2$, sowie 0,5 bis 1,5 Gew.-% NaOCl, vorzugsweise 1 Gew.-% NaOCl, eingesetzt werden.

8. Verfahren nach Anspruch 6, dadurch gekennzeichnet, daß die Bleiche ausschließlich mit NaOCl erfolgt, wobei 0,1 bis 3 Gew.-%, vorzugsweise 2 bis 3 Gew.-% des Bleichmittels — bezogen auf zu bleichendes Material — eingesetzt werden.

9. Verfahren nach Ansprüchen 6-8, dadurch gekennzeichnet, daß die Hypochlorit-Bleiche bei Temperaturen von etwa 50-70 °C, insbesondere im Bereich von 55-65 °C, durchgeführt wird, wobei vorzugsweise mit einer Bleichdauer im Bereich von 1 bis 10 Stunden, insbesondere im Bereich von 2 bis 4 Stunden, gearbeitet wird.

10. Verfahren nach Ansprüchen 1 bis 9, dadurch gekennzeichnet, daß die Abtrennung der begleitenden Glyzeride aus der Fettsäureesterfraktion nach deren Hydrierung vor der anschließenden Sulfonierung erfolgt.

11. Verfahren nach Ansprüchen 1 bis 10, dadurch gekennzeichnet, daß Fettsäureesterfraktionen mit einer Jod-Zahl unter 1, vorzugsweise unter 0,5, in die Sulfonierung eingesetzt werden.

12. Verfahren nach Ansprüchen 1 bis 11, dadurch gekennzeichnet, daß man wäßrige, neutralisierte Estersulfonatpasten mit Gehalten an Waschaktivsubstanz über 28 Gew.-%, insbesondere mit Feststoffgehalten im Bereich von 30-40 Gew.-%, herstellt.

13. Verfahren nach Ansprüchen 1 bis 12, dadurch gekennzeichnet, daß man mit gehärteten Talgfettsäuremethylestern und/oder gehärteten Palmölfettsäuremethylestern als Ausgangsmaterial für die Sulfonierung arbeitet.

### Claims

1. A process for the simplified recovery of light-colored washing-active esters of α-sulfo fatty acids or salts thereof from fats and oils of vegetable and/or animal origin by transesterification or hydrolysis and saponification of the fats or oils — if desired, hardened by hydrogenation — with monohydric $C_1$-$C_8$-alcohols, separation of the fatty acid ester fraction formed, hydrogenation thereof if necessary, subsequent sulfonation and bleaching of the crude sulfonation product, characterized in that a fatty acid ester fraction from which the accompanying fatty acid glycerides have been removed to a residual content of at most 1 % by weight, based on the material to be sulfonated, is introduced into the sulfonation step.

2. A process as claimed in Claim 1, characterized in that the residual content of the accompanying glycerides in the sulfonation step amounts to no more than about 0.5 % by weight and preferably to no more than about 0.3 % by weight.

3. A process as claimed in Claims 1 and 2, characterized in that, in the separation of the reaction mixture from the transesterification or hydrolysis and esterification of the fats and oils, a fatty acid ester fraction containing from about 2 to 5 % by weight and, more particularly, from about 2 to 3 % by weight of accompanying glycerides is recovered by distillation, freed from the greater part of the glycerides by redistillation and sulfonated.

4. A process as claimed in Claims 1 to 3, characterized in that the second distillation of the fatty acid ester fraction is carried out by overhead distillation at 1 mbar up to a sump temperature of around 280 °C.

5. A process as claimed in Claims 1 to 4, characterized in that sulfonation is carried out up to a degree of sulfonation of at least 92 % and preferably of at least 95 %.

6. A process as claimed in Claims 1 to 5, characterized in that the α-sulfo fatty acid ester crude product is bleached with hydrogen peroxide or $H_2O_2$ donors and/or hypochlorite to form products having a Klett color index of at most 60 and preferably of 50 or less, the crude product best being subjected (a) either to combined bleaching with $H_2O_2$ in the acidic range and then with hypochlorite in the pH-range of 6.5 to 10 or (b) solely to hypochlorite bleaching in the pH-range of 6.5 to 10.

7. A process as claimed in Claim 6, characterized in that from 0.5 to 1.5 % by weight of $H_2O_2$, expressed as 100 % $H_2O_2$ and based on the sulfonated material, and preferably 1 % by weight of $H_2O_2$ and from 0.5 to 1.5 % by weight of NaOCl, preferably 1 % by weight of NaOCl are used in the combined bleaching treatment (a).

8. A process as claimed in Claim 6, characterized in that bleaching is carried out solely with NaOCl used in a quantity of from 0.1 to 3 % by weight and preferably in a quantity of from 2 to 3 % by weight, based on the material to be bleached.

9. A process as claimed in Claims 6 to 8, characterized in that the bleaching with hypochlorite is carried out at temperatures in the range from about 50 to 70 °C and more particularly in the range from 55 to 65 °C and over a period of from 1 to 10 hours and more particularly over a period of from 2 to 4 hours.

10. A process as claimed in Claims 1 to 9, characterized in that separation of the accompanying glycerides from the fatty acid ester fraction is carried out after its hydrogenation and before the subsequent sulfonation step.

11. A process as claimed in Claims 1 to 10, characterized in that fatty acid ester fractions having an iodine number below 1 and preferably below 0.5 are subjected to the sulfonation step.

12. A process as claimed in Claims 1 to 11, characterized in that aqueous, neutralized ester sulfonate pastes having contents of washing-active substance of more than 28 % by weight and, more particularly, solids contents of from 30 to 40 % by weight, are produced.

13. A process as claimed in Claims 1 to 12, characterized in that hardened tallow fatty acid methyl esters and/or hardened palm oil fatty acid methyl esters are used as starting material for the sulfonation step.

**Revendications**

1. Procédé pour simplifier l'obtention d'esters alpha-sulfoniques d'acides gras tensio-actifs de couleurs claires ou de leurs sels à partir de graisses ou d'huiles d'origine végétale et/ou animale, par transestérification ou saponification et estérification des graisses ou huiles éventuellement durcies par hydrogénation, avec des alcools monovalents en $C_1$ à $C_8$, séparation des fractions esters d'acides gras formées, leur hydrogénation si nécessaire, sulfonation subséquente et décoloration des produits bruts de sulfonation, procédé caractérisé en ce que dans l'étape de sulfonation on introduit une fraction ester d'acide gras d'où les glycérides d'acide gras d'accompagnement ont été éliminés jusqu'à une teneur résiduelle ne dépassant pas 1 % en poids, calculée sur la matière à sulfoner.

2. Procédé selon la revendication 1, caractérisé en ce que la teneur résiduelle en glycéride d'accompagnement dans l'étape de sulfonation ne dépasse pas environ 0,5 % en poids, de préférence environ 0,3 % en poids.

3. Procédé selon l'une des revendications 1 ou 2, caractérisé en ce que lors de la séparation du mélange réactionnel de la transestérification ou de la saponification et estérification de la graisse ou huile, on obtient par distillation une fraction ester d'acide gras avec une teneur en glycéride d'accompagnement d'environ 2 à 5 % en poids, en particulier d'environ 2 à 3 % en poids, que par renouvellement de la distillation, on élimine de cette fraction la majeure partie des glycérides, et qu'elle est soumise à la sulfonation.

4. Procédé selon les revendications 1 à 3, caractérisé en ce que la deuxième distillation de la fraction ester d'acide gras s'effectue par une distillation « en tête » jusqu'à une température du fond d'environ 280 °C sous 1 mbar.

5. Procédé selon les revendications 1 à 4, caractérisé en ce que la sulfonation s'effectue jusqu'à un degré de sulfonation d'au moins 92 %, de préférence au moins 95 %.

6. Procédé selon les revendications 1 à 5, caractérisé en ce que le produit brut ester d'acide gras α-sulfoné est soumis à une décoloration avec du peroxyde d'hydrogène et/ou des composés libérants du $H_2O_2$ et/ou de l'hypochlorite jusqu'à obtention d'un produit présentant un indice de coloration selon Klett d'au plus 60, de préférence 50 au moins, les opérations effectuées ici de préférence étant : a) décoloration combinée avec $H_2O_2$ en milieu acide suivie d'un traitement à l'hypochlorite dans une zone de pH de 6,5 à 10, ou b) traitement exclusif ou hypochlorite dans une zone de pH de 6,5 à 10.

7. Procédé selon la revendication 6, caractérisé en ce que dans la décoloration combinée selon (a), on met en œuvre 0,5 à 1,5 % en poids de $H_2O_2$ — calculé en $H_2O_2$ à 100 % et rapporté à la matière à sulfoner — de préférence 1 % en poids de $H_2O_2$ ainsi que 0,5 à 1,5 % en poids de NaOCl, de préférence 1 % en poids de NaOCl.

8. Procédé selon la revendication 6, caractérisé en ce que la décoloration s'effectue exclusivement avec NaOCl, avec la mise en œuvre de 0,1 à 3 % en poids, de préférence 2 à 3 % en poids de l'agent décolorant calculé sur la matière à décolorer.

9. Procédé selon les revendications 6 à 8, caractérisé en ce que la décoloration à l'hypochlorite s'effectue à une température de 50 à 70 °C environ, en particulier de 55 à 65 °C, en opérant de préférence avec une durée de décoloration d'environ 1 à 10 heures, de préférence 2 à 4 heures.

10. Procédé selon les revendications 1 à 9, caractérisé en ce que la séparation des glycérides d'accompagnement de la fraction ester d'acide gras s'effectue après l'hydrogénation de cette fraction et avant sa sulfonation subséquente.

11. Procédé selon les revendications 1 à 10, caractérisé en ce que des fractions d'ester d'acide gras ayant un indice d'iode inférieur à 1, de préférence inférieur à 0,5, sont mises en œuvre pour la sulfonation.

12. Procédé selon les revendications 1 à 11, caractérisé en ce que l'on produit des pâtes de sulfonates d'ester neutralisés, avec une teneur en substances tensio-actives supérieure à 28 % en poids, en particulier avec une teneur en matière solide comprise entre 30 et 40 % en poids.

13. Procédé selon les revendications 1 à 12, caractérisé en ce que comme matière de départ pour la sulfonation, on utilise des esters méthyliques d'acide stéarique solidifiés, et/ou des esters méthyliques d'acide palmitique solidifiés.